# EUROPEAN PATENT APPLICATION

(11) **EP 2 997 888 A1**
(43) Date of publication of application: **23.03.2016**
(21) Application number: 15185689.5
(22) Date of filing: 17.09.2015
(51) Int. Cl.: A61B 5/00, A61B 5/1455, A61B 5/1459, A61B 18/14, A61B 17/00

(54) **MULTI-RANGE OPTICAL SENSING DEVICE**

(30) Priority: 18.09.2014 US 201414490210
(71) Applicant: Biosense Webster (Israel) Ltd., 2066717 Yokneam (IL)
(72) Inventor: BEECKLER, Christopher Thomas, Brea, CA 92821 (US); KEYES , Joseph Thomas, Glendora, CA 91741 (US)
(74) Representative: Carpmaels & Ransford LLP

(57) **Abstract**

The depth of an ablation lesion is assessed using a differential optical response of a catheter with multiple fiberoptic transmitters and receivers at the tip. To detect tissue optical response at shallow depths, closely-spaced transmitter/receiver pairs of optical fibers are used. To detect deeper tissue response, the same or a different transmitter can be used with another receiver that is relatively farther away. The distance between the transmitter and receiver is chosen depending on the desired depth of sensing. Plateauing or peaking of the optical signal during the course of ablation indicates an end point at a selected tissue depth.

## Description

### BACKGROUND OF THE INVENTION

### Field of the Invention

This invention relates to invasive medical devices. More particularly, this invention relates to ablation of tissue using such devices.

### Description of the Related Art

Ablation of body tissue using electrical energy is known in the art. The ablation is typically performed by applying alternating currents, for example radiofrequency energy, to the electrodes, at a sufficient power to destroy target tissue. Typically, the electrodes are mounted on the distal tip of a catheter, which is inserted into a subject. The distal tip may be tracked in a number of different ways known in the art, for example by measuring magnetic fields generated at the distal tip by coils external to the subject.

A known difficulty in the use of radiofrequency energy for cardiac tissue ablation is controlling local heating of tissue. There are tradeoffs between the desire to create a sufficiently large lesion to effectively ablate an abnormal tissue focus, or block an aberrant conduction pattern, and the undesirable effects of excessive local heating. If the radiofrequency device creates too small a lesion, then the medical procedure could be less effective, or could require too much time. On the other hand, if tissues are heated excessively then there could be local charring effects, coagulum, and or explosive steam pops due to overheating. Such overheated areas can develop high impedance, and may form a functional barrier to the passage of heat. The use of slower heating provides better control of the ablation, but unduly prolongs the procedure.

U.S. Patent No. 8,147,484 to Lieber et al. discloses real-time optical measurements of tissue reflection spectral characteristics while performing ablation. The technique involves the radiation of tissue and recapturing of light from the tissue to monitor changes in the reflected optical intensity as an indicator of steam formation in the tissue for prevention of steam pop. Observation is made to determine whether measured reflectance spectral intensity (MRSI) increases during a specified time period followed by a decrease at a specified rate in the MRSI. If there is a decrease in the MRSI within a specified time and at a specified rate, then formation of a steam pocket is inferred.

### SUMMARY OF THE INVENTION

Commonly assigned U.S. Provisional Application No. 61/984953, which is herein incorporated by reference, discloses that optical reflectivity measured by optical sensors near the tip of a catheter indicate events, such as imminent occurrence of steam pops.

According to disclosed embodiments of the invention, the depth of an ablation lesion is assessed using a differential optical response of a catheter with multiple fiberoptic transmitters and receivers at the tip. To detect tissue optical response at shallow depths, closely-spaced transmitter/receiver pairs are used. To detect deeper tissue response, the same transmitter can be used with another receiver that is farther away (or vice versa). The distance between the transmitter and receiver is chosen depending on the desired depth of sensing. Plateauing or peaking of the optical signal during the course of ablation indicates an end point at a selected tissue depth.

There is provided according to embodiments of the invention an insertion tube configured for insertion into proximity with tissue in a body of a patient. The tube has an electrical conductor for delivering energy to the tissue and a conductive cap attached to the distal portion of the insertion tube and coupled electrically to the electrical conductor. A plurality of optical fibers contained within the insertion tube have terminations at the distal portion. The optical fibers are configurable as optical transmitting fibers to convey optical radiation to the tissue and as optical receiving fibers to convey reflected optical radiation from the tissue. At the distal portion of the insertion tube, the terminations of the optical fibers are spaced apart at respective distances from one another. An optical module is configured to interrogate the tissue at a predetermined depth by selectively associating the optical transmitting fibers with the optical receiving fibers according to the respective distances therebetween, the optical module being operative to emit light along a light path that passes through a selected optical transmitting fiber, reflects from the tissue, and returns to the optical module as reflected light via a selected optical receiving fiber while the electrical conductor is delivering energy to the tissue. A processor linked to the optical module analyzes the reflected light.

According to another aspect of the apparatus, the optical module is operative for varying an intensity of the light that is emitted in the light path.

According to still another aspect of the apparatus, the emitted light in the light path is monochromatic.

According to an additional aspect of the apparatus, the emitted light in the light path has a wavelength of 675 nm.

According to another aspect of the apparatus, the selectively associated optical transmitting fibers and optical receiving fibers are spaced apart by intervals of 0.5 - 2 mm.

According to one aspect of the apparatus, analyzing the reflected light includes determining a time at which the reflected light ceases to vary in intensity by more than a predetermined rate.

According to a further aspect of the apparatus, analyzing the reflected light includes identifying a time of a peak in intensity in the returning light.

According to still another aspect of the apparatus, analyzing the reflected light includes determining at respective depths of interrogation times at which variations in a rate of change of a reflected light intensity by more than a predetermined percentage occur.

According to an additional aspect of the apparatus, analyzing the reflected light includes calculating a ratio of two wavelengths and determining a time at which the ratio ceases to vary by more than a predetermined rate.

There is further provided according to embodiments of the invention a method, which is carried out by configuring optical fibers contained within a probe as optical transmitting fibers and as optical receiving fibers, wherein terminations of the optical fibers are spaced apart at respective distances from one another, inserting the probe into a body of a patient. While delivering energy to a tissue in the body through an ablator of the probe, the method is further carried out by interrogating the tissue at a predetermined depth by selectively associating one of the optical transmitting fibers with one of the optical receiving fibers according to the respective distances therebetween, and establishing a light path extending from a light emitter through the one optical transmitting fiber to reflect from the tissue and continuing as reflected light from the tissue through the one optical receiving fiber to a receiver. The method is further carried out by transmitting light from the light emitter along the light path, and analyzing the reflected light reaching the receiver via the one optical receiving fiber.

### BRIEF DESCRIPTION OF THE SEVERAL VIEWS OF THE DRAWINGS

For a better understanding of the present invention, reference is made to the detailed description of the invention, by way of example, which is to be read in conjunction with the following drawings, wherein like elements are given like reference numerals, and wherein:
Fig. 1 is a pictorial illustration of a system for performing ablative procedures, which is constructed and operative in accordance with a disclosed embodiment of the invention;
Fig. 2 is a schematic, perspective illustration of a catheter cap in accordance with an embodiment of the invention;
Fig. 3 is an isometric view of the distal end of a catheter in accordance with an alternate embodiment of the invention;
Fig. 4 is a schematic side view taken along line 5-5 of Fig. 4, in accordance with an embodiment of the invention;
Fig. 5 schematically illustrates paths taken by light to/from windows in the cap shown in Fig. 2, in accordance with an embodiment of the invention;
Fig. 6 is a schematic view of the distal end of a catheter, in accordance with an embodiment of the invention;
Fig. 7 is a plot that relates the inter-element distance of an optical receiver-transmitter pair in a catheter to the elapsed time at which an ablation endpoint is observed, in accordance with an embodiment of the invention; and
Fig. 8 is a series of plots showing the effect of varying the intensity of optical radiation, in accordance with an embodiment of the invention.

### DETAILED DESCRIPTION OF THE INVENTION

In the following description, numerous specific details are set forth in order to provide a thorough understanding of the various principles of the present invention. It will be apparent to one skilled in the art, however, that not all these details are necessarily needed for practicing the present invention. In this instance, well-known circuits, control logic, and the details of computer program instructions for conventional algorithms and processes have not been shown in detail in order not to obscure the general concepts unnecessarily.

### Overview

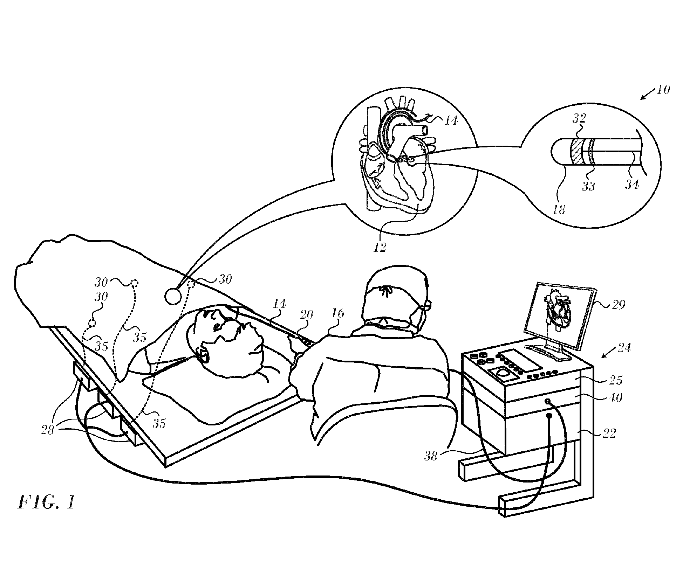

Turning now to the drawings, reference is initially made to Fig. 1, which is a pictorial illustration of a system 10 for evaluating electrical activity and performing ablative procedures on a heart 12 of a living subject, which is constructed and operative in accordance with a disclosed embodiment of the invention. The system comprises a catheter 14, which is percutaneously inserted by an operator 16 through the patient's vascular system into a chamber or vascular structure of the heart 12. The operator 16, who is typically a physician, brings the catheter's distal tip 18 into contact with the heart wall, for example, at an ablation target site. Electrical activation maps may be prepared, according to the methods disclosed in U.S. Patent Nos. 6,226,542, and 6,301,496, and in commonly assigned U.S. Patent No. 6,892,091, whose disclosures are herein incorporated by reference. One commercial product embodying elements of the system 10 is available as the CARTO^{®} 3 System, available from Bio-sense Webster, Inc., 3333 Diamond Canyon Road, Diamond Bar, CA 91765. This system may be modified by those skilled in the art to embody the principles of the invention described herein.

Areas determined to be abnormal, for example by evaluation of the electrical activation maps, can be ablated by application of thermal energy, e.g., by passage of radiofrequency electrical current through wires in the catheter to one or more electrodes at the distal tip 18, which apply the radiofrequency energy to the myocardium. The energy is absorbed in the tissue, heating it to a point (typically above 50°C) at which it permanently loses its electrical excitability. When successful, this procedure creates non-conducting lesions in the cardiac tissue, which disrupt the abnormal electrical pathway causing the arrhythmia. The principles of the invention can be applied to different heart chambers to diagnose and treat many different cardiac arrhythmias.

The catheter 14 typically comprises a handle 20, having suitable controls on the handle to enable the operator 16 to steer, position and orient the distal end of the catheter as desired for the ablation. To aid the operator 16, the distal portion of the catheter 14 contains position sensors (not shown) that provide signals to a processor 22, located in a console 24. The processor 22 may fulfill several processing functions as described below.

Ablation energy and electrical signals can be conveyed to and from the heart 12 through one or more ablation electrodes 32 located at or near the distal tip 18 via cable 34 to the console 24. Pacing signals and other control signals may be conveyed from the console 24 through the cable 34 and the electrodes 32 to the heart 12. Sensing electrodes 33, also connected to the console 24 are disposed between the ablation electrodes 32 and have connections to the cable 34.

Wire connections 35 link the console 24 with body surface electrodes 30 and other components of a positioning sub-system for measuring location and orientation coordinates of the catheter 14. The processor 22 or another processor (not shown) may be an element of the positioning subsystem. The electrodes 32 and the body surface electrodes 30 may be used to measure tissue impedance at the ablation site as taught in U.S. Patent No. 7,536,218, issued to Govari et al., which is herein incorporated by reference. A temperature sensor (not shown), typically a thermocouple or thermistor, may be mounted on or near each of the electrodes 32.

The console 24 typically contains one or more ablation power generators 25. The catheter 14 may be adapted to conduct ablative energy to the heart using any known ablation technique, e.g., radiofrequency energy, ultrasound energy, and laser-produced light energy. Such methods are disclosed in commonly assigned U.S. Patent Nos. 6,814,733, 6,997,924, and 7,156,816, which are herein incorporated by reference.

In one embodiment, the positioning subsystem comprises a magnetic position tracking arrangement that determines the position and orientation of the catheter 14 by generating magnetic fields in a predefined working volume and sensing these fields at the catheter, using field generating coils 28. The positioning subsystem is described in U.S. Patent No. 7,756,576, which is hereby incorporated by reference, and in the above-noted U.S. Patent No. 7,536,218.

As noted above, the catheter 14 is coupled to the console 24, which enables the operator 16 to observe and regulate the functions of the catheter 14. Console 24 includes a processor, preferably a computer with appropriate signal processing circuits. The processor is coupled to drive a monitor 29. The signal processing circuits typically receive, amplify, filter and digitize signals from the catheter 14, including signals generated by sensors such as electrical, temperature and contact force sensors, and a plurality of location sensing electrodes (not shown) located distally in the catheter 14. The digitized signals are received and used by the console 24 and the positioning system to compute the position and orientation of the catheter 14, and to analyze the electrical signals from the electrodes.

In order to generate electroanatomic maps, the processor 22 typically comprises an electroanatomic map generator, an image registration program, an image or data analysis program and a graphical user interface configured to present graphical information on the monitor 29.

An optical module 40 provides optical radiation, typically from, but not limited to, a laser, an incandescent lamp, an arc lamp, or a light emitting diode (LED), for transmission from distal tip 18 to the target tissue. The module receives and cooperatively with the processor 22 analyzes optical radiation returning from the target tissue and acquired at the distal end, as described below.

Typically, the system 10 includes other elements, which are not shown in the figures for the sake of simplicity. For example, the system 10 may include an electrocardiogram (ECG) monitor, coupled to receive signals from one or more body surface electrodes, in order to provide an ECG synchronization signal to the console 24. As mentioned above, the system 10 typically also includes a reference position sensor, either on an externally-applied reference patch attached to the exterior of the subject's body, or on an internally-placed catheter, which is inserted into the heart 12 maintained in a fixed position relative to the heart 12. Conventional pumps and lines for circulating liquids through the catheter 14 for cooling the ablation site are provided. The system 10 may receive image data from an external imaging modality, such as an MRI unit or the like and includes image processors that can be incorporated in or invoked by the processor 22 for generating and displaying images.

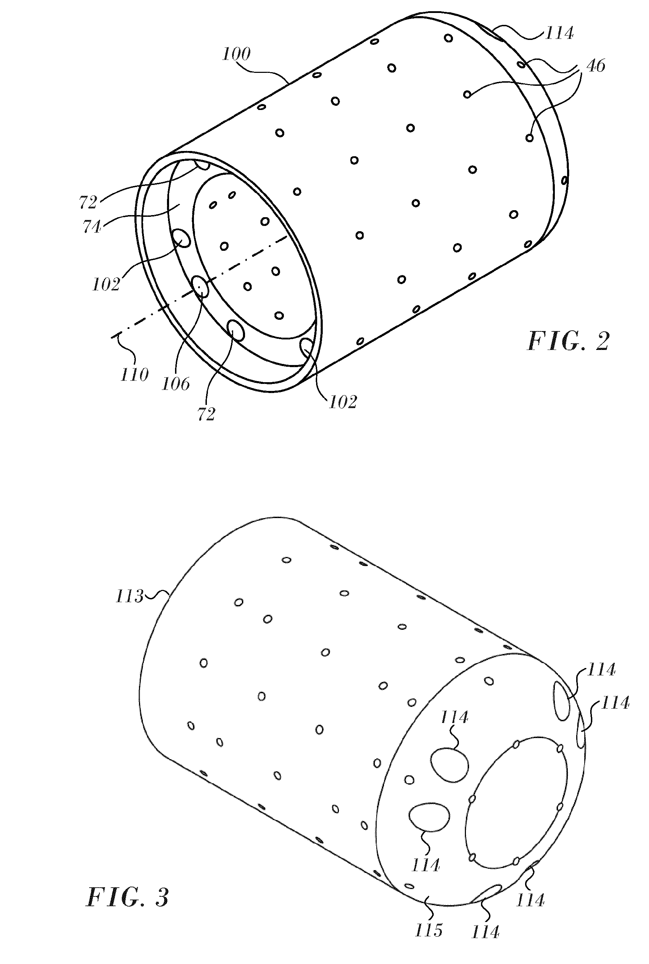

Reference is now made to Fig. 2, which is a schematic, perspective illustration of a catheter cap 100, in accordance with an embodiment of the invention. Cap 100 comprises a side wall 74 that is on the order of 0.4 mm thick, in order to provide the desired thermal insulation between optional temperature sensors 48 and the irrigation fluid inside a central cavity 76 of the tip. Irrigation fluid exits cavity 76 through apertures 46.

Reference is now made to Fig. 3, which is an isometric view of the distal end of a cap 113 for a catheter in accordance with an alternate embodiment of the invention. In this embodiment six openings 114 are located at distal end 115. As explained below the opening 114 constitute windows at the terminations of fiberoptic elements that extend longitudinally through the catheter 14 into the cap 113. In other embodiments, the cap 113 may be provided with other windows (not shown) to accommodate sensors, e.g., temperature or contact force sensors.

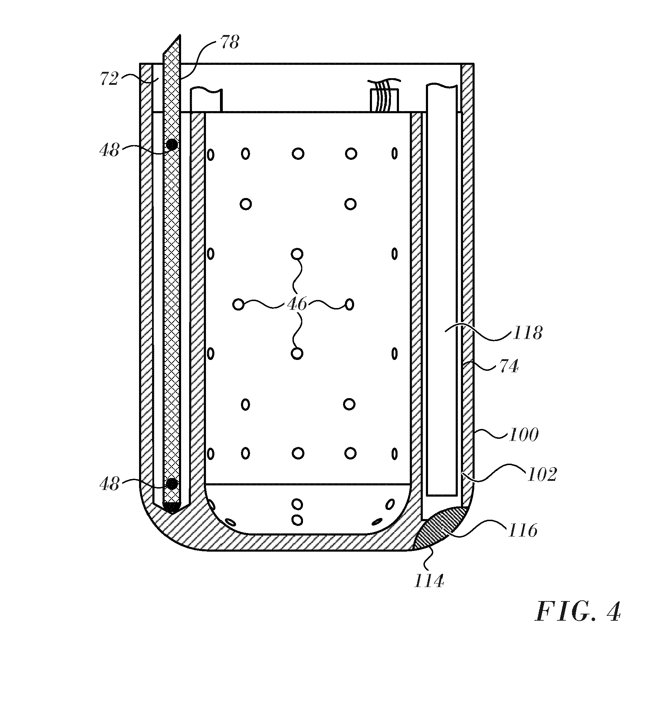

Reference is now made to Fig. 4, which is a schematic side view showing the interior of the cap 100 (Fig. 2), in accordance with an embodiment of the invention. Three through longitudinal bores 102 and three blind longitudinal bores 72 are formed in side wall 74. The three sets of bores 72, 102 may be distributed symmetrically around a longitudinal axis of cap 100. However, the bores are not necessarily distributed symmetrically around the axis. Optional sensors 48 are mounted in hollow tubes 78, which are filled with a suitable glue, such as epoxy and fitted into longitudinal bores 72 in side wall 74. Tubes 78 may comprise a suitable plastic material, such as polyimide, and may be held in place by a suitable glue, such as epoxy. This arrangement provides an array of sensors 48, with possible advantages of greater ease of manufacture and durability.

Each through longitudinal bore 102 terminates in an opening 114 in the surface of wall 74, and a transparent window 116 is placed in the opening. A fiber optic 118 is inserted into each of the through bores. In some embodiments, temperature sensors 48 may not be installed, and only fiber optics 118 are incorporated into the wall. Such an embodiment enables determination of tissue contact with the cap, and/or characterization of the tissue in proximity to the cap, by methods described below.

Window 116 acts as a seal preventing fluid external to the outer surface of cap 100 from penetrating into the bores containing the fiber optics. Window 116 may be formed by filling opening 114 with an optically transparent glue or epoxy. In some embodiments, the material of the windows may be filled with a scattering agent to diffuse light passing through the windows.

Alternatively, the windows may be formed from an optical quality flat or lensed material, and may be secured to their openings with glue.

In one embodiment, each fiber optic 118 or each fiber optic 128 is a single fiber optic, typically having a diameter of approximately 175 µm. In an alternative embodiment each fiber optic 118 or each fiber optic 128 comprises a bundle of substantially similar fiber optics, typically having a bundle diameter also of approximately 175 µm. Implementing the fiber optics as bundles increases the flexibility of cap 100 with respect to more proximal regions of the catheter 14 (Fig. 1).

Such an increase in flexibility is advantageous if cap 100 is connected to the more proximal regions of the catheter by a spring whose deflections are measured for the purpose of measuring a force on the cap, since the increased flexibility means there is little or no change in the spring deflection for a given force. A spring that may be used to join the cap 100 to the more proximal regions of the catheter is described in U.S. Patent Application Publication No. 2011/0130648 by Beeckler et al., whose disclosure is incorporated herein by reference.

Optical module 40 (Fig. 1) is configured to be able to provide optical radiation to any one of fiber optics 118 and 128, for transmission from any of the associated windows 116, 124 in order to irradiate tissue in proximity to cap 100. Simultaneously, the optical module 40 is able to acquire, via any or all of the windows, radiation returning from the irradiated tissue.

The array of windows 116, 124, and their associated fiber optics, enables embodiments of the present invention to employ a number of different methods, using optical radiation, for determining characteristics of the irradiated tissue, as well as the proximity of cap 100, or a region of the cap, with respect to the tissue. By way of example, three such methods are described below, but those having ordinary skill in the art will be aware of other methods, and all such methods are included within the scope of the present invention.

A first method detects contact of any one of windows 116, 124, and consequently of the catheter, with tissue. Optical radiation, of a known intensity, is transmitted through each fiber optic, to exit from the optic's window. The intensity of the radiation returning to the window is measured while cap 100 is not in contact with tissue, typically while the cap is in the blood of heart 12 (Fig. 1). Optical module 40 may use these intensities as reference values of the optical radiation.

For any given window, a change in the value from the window's reference value, as measured by the module, may be taken to indicate that the window is in contact with tissue.

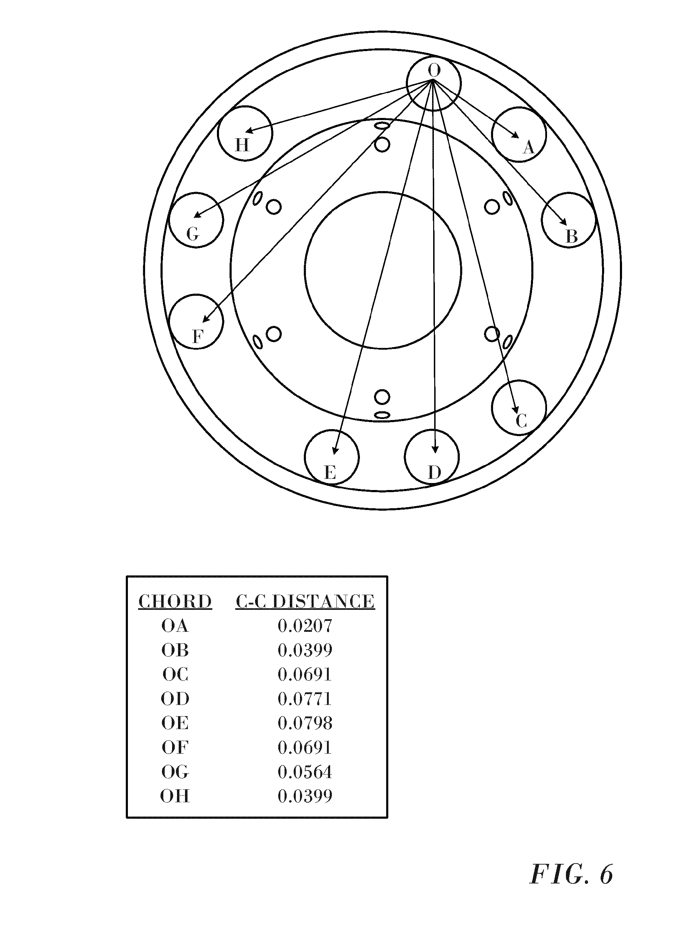

A second method measures characteristics of tissue being irradiated by the optical radiation. Reference is now made to Fig. 6, which schematically illustrates paths taken by light to/from windows in the cap 100 (Fig. 2), in accordance with an embodiment of the invention.

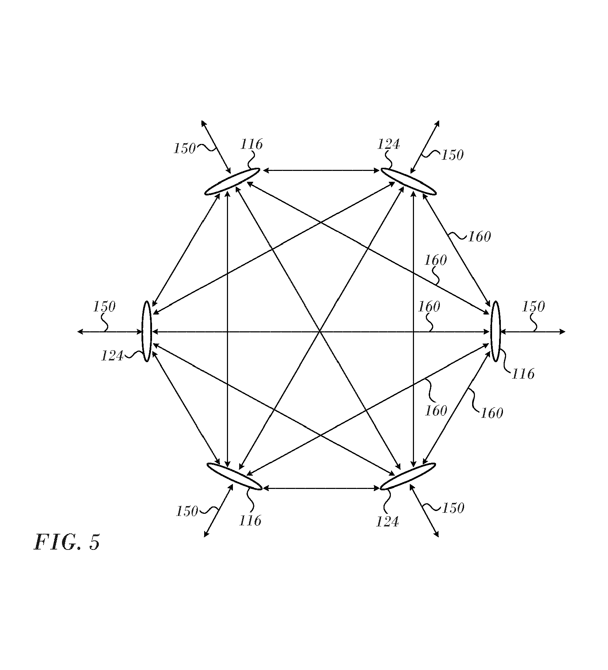

As illustrated in Fig. 5, for all six windows 116, 124 there are a total of 21 different paths, comprising 6 paths 150 where radiation from a given window returns to that window, and 15 paths 160 where radiation from a given window returns to a different window. The change of optical radiation for a given path or group of paths depends on characteristics of tissue in the path or group of paths, so that measurements of the change in all of the paths provide information related to characteristics of the tissue in proximity to cap 100.

For example, the change in all of the paths may be measured by sequentially transmitting, in a time multiplexed manner, optical radiation from each of the windows 116, 124, and measuring the returning radiation. A first transmission from a first window in such a sequence provides values for five paths 160 plus a return path 150 to the first window. A second transmission from a second window provides values for four new paths 160 plus return path 150 to the second window. A third transmission from a third window provides values for three new paths 160 plus return path 150 to the third window. A fourth transmission from a fourth window provides values for two new paths 160 plus return path 150 to the fourth window. A fifth transmission from a fifth window provides values for one new path 160 to the sixth window, and return path 150 to the fifth window). A sixth and final transmission from a sixth window provides one return path 150 through the sixth window.

Optical module 40 (Fig. 1) enables a first portion of the fibers as optical transmitting fibers and a second portion of the fibers as optical receiving fibers. The optical module 40 selectively associates the optical transmitting fibers with the optical receiving fibers to produce a light path passing through a selected optical transmitting fiber, reflecting from the target tissue, and returning via a selected optical receiving fiber. As the first portion and the second portion of the fibers are spaced apart at respective distances, by appropriate choice of an optical transmitting fiber and an optical receiving fiber, the optical module 40 is able to interrogate the target tissue at a desired depth according to inter-element spacing between the optical transmitting fiber and the optical receiving fiber. The optical module 40 cooperatively with the processor 22 (Fig. 1) may measure the changes of all the paths, and, using a calibration procedure, may derive from the changes optical characteristics of tissue within the paths. Such characteristics may include an overall level of ablation of tissue, or an amount and/or type of necrotic tissue, in the paths.

The light in the light path may be monochromatic light, for example at a wavelength of 675 nm. Alternatively, the light may have broader spectrum.

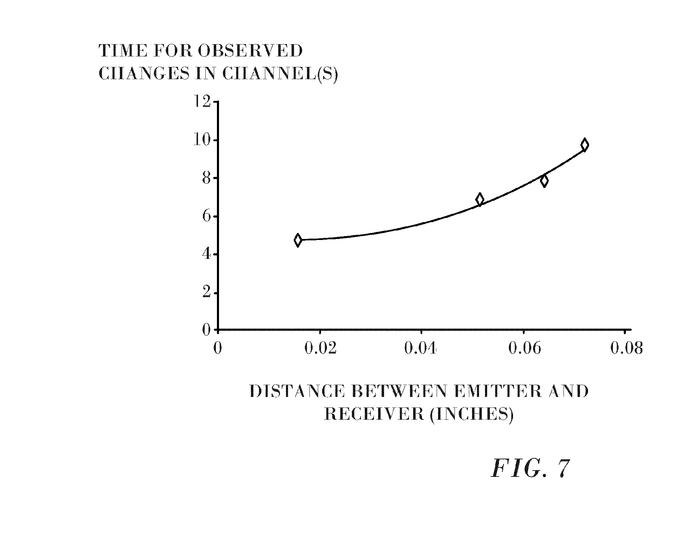

Reference is now made to Fig. 6, which is a schematic view of the distal end of a catheter, in accordance with an embodiment of the invention. Nine terminations of fiberoptic elements (O, A - H) are shown. Chords OA-OH connect element O with elements A-H, respectively. The accompanying table indicates the corresponding inter-element distances of the terminations. While Fig. 7 exhaustively depicts light paths in respect of element O, in practice not all of the positions need be dedicated to fiberoptic elements. In a current embodiment, three positions (elements H, B, and E) are assigned to temperature sensors, thereby leaving fewer light paths to be selected,

### Operation

Continuing to refer to Fig. 6, the catheter is operated in cooperation with the system 10 (Fig. 1) by configuring an element, e.g., element O, as one member of an optical receiver-transmitter pair and another element, e.g., elements A-H as the other member. The selected receiver-transmitter pair is optimum for interrogating the ablation site at a respective depth. For example, an inter-element distance of 0.5 mm is optimum for a shallow depth of interrogation. An inter-element distance of about 2 mm is optimum for a deeper level of approximately 2-3mm. The selected inter-element distance may be varied, either by holding one element, e.g., element O, fixed, and analyzing the other elements in turn, or by changing the pairing according to a predetermined schedule. In any case, the reflectances measured by the pairs are analyzed as the ablation proceeds. Once the signal is received using the largest inter-element distance stabilizes (or peaks), it may be concluded that no further changes are occurring in the tissue at that level. Although the optical interrogation depth is approximately 2-3 mm, the total depth of the lesion can be extrapolated based upon the magnitude of change at the maximum interrogation depth. Alternatively, by operating a plurality of the elements as optical transmitters at respective wavelengths, multiple receiver-transmitter pairs may be operated concurrently.

### Results

Reference is now made to Fig. 7, which is a plot that relates the inter-element distance of optical receiver-transmitter pairs in a catheter to the elapsed time at which a change in optical intensity is observed, in accordance with an embodiment of the invention. In a medical procedure of this sort, the depth of ablation increases with elapsed time. A correlation is shown between the interrogation depth at a particular distance and the elapsed time, indicating that optical reflectances at increasing receiver-transmitter pair distances are useful for detecting increasing ablation depths.

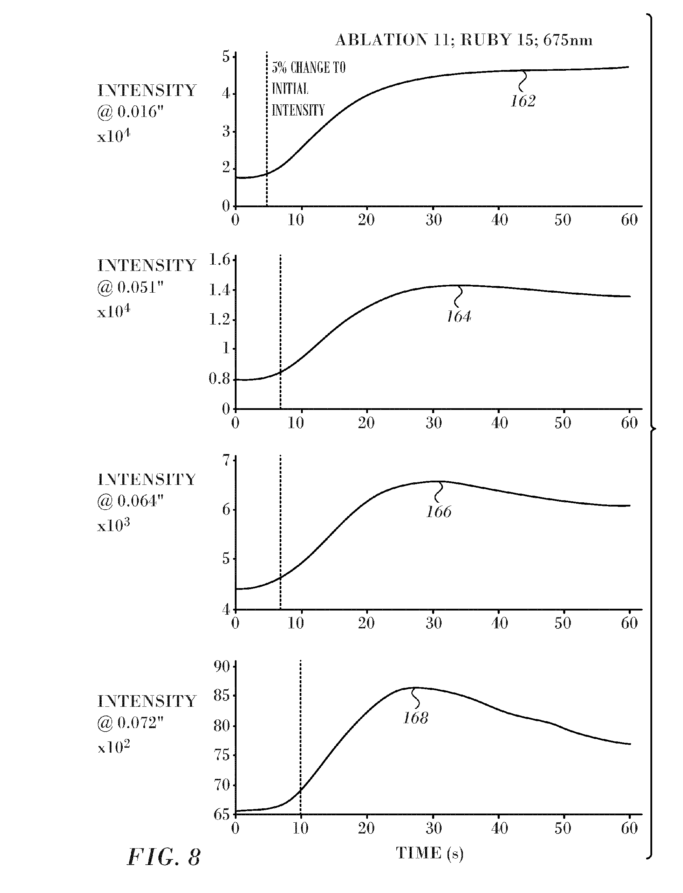

Reference is now made to Fig. 8, which is a series of plots showing the effect of varying the intensity of optical radiation, in accordance with an embodiment of the invention. An endpoint may be determined by establishing a time at which the intensity of the reflected light fails to vary by more than a predetermined rate. Alternatively, the endpoint may be determined by identification of a peak in the intensity of the reflected light endpoints 162, 162, 164, 166.

Alternatively, the endpoint may be determined by transmitting light through a path via the fiberoptic elements at two wavelengths and calculating a ratio of the reflected light at the two wavelengths. The endpoint may be defined as a time at which the ratio ceases to vary by more than a predetermined rate.

Analysis of reflectance data may comprise identification of a point (referred to herein as a "startpoint"). As the interrogation depth increases, startpoints represents times at which variations in the rate of change of reflectance by more than a predetermined percentage occur. Such startpoints correspond respectively to different interrogation depths. The first startpoints occur at shallow interrogation depths and the later instances occur at deeper interrogation depths.

The lowermost plot was obtained using the highest separation distance, and exhibits a distinct peak, whereas lower separation distances result in a flattening or plateau after an endpoint of the ablation is reached as shown by points 162, 164, 166, 168.

It will be appreciated by persons skilled in the art that the present invention is not limited to what has been particularly shown and described hereinabove. Rather, the scope of the present invention includes both combinations and sub-combinations of the various features described hereinabove, as well as variations and modifications thereof that are not in the prior art, which would occur to persons skilled in the art upon reading the foregoing description.

### Aspects of the invention:

1. A method, comprising the steps of:
   configuring optical fibers contained within a probe as optical transmitting fibers and as optical receiving fibers, wherein terminations of the optical fibers are spaced apart at respective distances from one another;
   inserting the probe into a body of a patient;
   while delivering energy to a tissue in the body through an ablator of the probe, interrogating the tissue at a predetermined depth by selectively associating one of the optical transmitting fibers with one of the optical receiving fibers according to the respective distances there between; and
   establishing a light path extending from a light emitter through the one optical transmitting fiber to reflect from the tissue and continuing as reflected light from the tissue through the one optical receiving fiber to a receiver;
   transmitting light from the light emitter along the light path; and
   analyzing the reflected light reaching the receiver via the one optical receiving fiber.
2. The method according to aspect 1, wherein transmitting light comprises varying an intensity of the transmitted light.
3. The method according to aspect 1, wherein the light emitter emits monochromatic light.
4. The method according to aspect 3, wherein the light emitter emits light having a wavelength of 675 nm.
5. The method according to aspect 1, wherein the selectively associated optical transmitting fibers and optical receiving fibers are spaced apart by intervals of 0.5 - 2 mm.
6. The method according to aspect 1, comprising operating a plurality of receiver-transmitter pairs of the optical fibers concurrently at respective wavelengths.
7. The method according to aspect 1, wherein analyzing the reflected light comprises determining a time at which the reflected light ceases to vary in intensity by more than a predetermined rate.
8. The method according to aspect 1, wherein analyzing the reflected light comprises identifying a time of a peak in intensity in the reflected light.
9. The method according to aspect 1, wherein analyzing the reflected light comprises determining at respective depths of interrogation times at which variations in a rate of change of a reflected light intensity by more than a predetermined percentage occur.
10. The method according to aspect 1, wherein analyzing the reflected light comprises calculating a ratio of two wavelengths and determining a time at which the ratio ceases to vary by more than a predetermined rate.

## Claims

1. An apparatus, comprising:
an insertion tube having a distal portion configured for insertion into proximity with tissue in a body of a patient and containing a lumen comprising:
an electrical conductor for delivering energy to the tissue;
a conductive cap attached to the distal portion of the insertion tube and coupled electrically to the electrical conductor;
a plurality of optical fibers contained within the insertion tube and having terminations at the distal portion, the optical fibers being configurable as optical transmitting fibers to convey optical radiation to the tissue and being configurable as optical receiving fibers to convey reflected optical radiation from the tissue, wherein at the distal portion of the insertion tube, the terminations of the optical fibers are spaced apart at respective distances from one another;
an optical module configured to interrogate the tissue at a predetermined depth by selectively associating the optical transmitting fibers with the optical receiving fibers according to the respective distances therebetween, the optical module operative to emit light along a light path that passes through a selected optical transmitting fiber, reflects from the tissue, and returns to the optical module as reflected light via a selected optical receiving fiber while the electrical conductor is delivering energy to the tissue; and
a processor linked to the optical module for analyzing the reflected light.

2. The apparatus according to claim 1, wherein the optical module is operative for varying an intensity of the light being emitted in the light path.

3. The apparatus according to claim 1, wherein the emitted light in the light path is monochromatic.

4. The apparatus according to claim 3, wherein the emitted light in the light path has a wavelength of 675 nm.

5. The apparatus according to claim 1, wherein the selectively associated optical transmitting fibers and optical receiving fibers are spaced apart by intervals of 0.5 - 2 mm.

6. The apparatus according to claim 1, wherein analyzing the reflected light comprises determining a time at which the reflected light ceases to vary in intensity by more than a predetermined rate.

7. The apparatus according to claim 1, wherein analyzing the reflected light comprises identifying a time of a peak in intensity in the returning light.

8. The apparatus according to claim 1, wherein analyzing the reflected light comprises determining at respective depths of interrogation times at which variations in a rate of change of a reflected light intensity by more than a predetermined percentage occur.

9. The apparatus according to claim 1, wherein analyzing the reflected light comprises calculating a ratio of two wavelengths and determining a time at which the ratio ceases to vary by more than a predetermined rate.
